# EUROPEAN PATENT APPLICATION

(11) **EP 4 442 711 A1**
(43) Date of publication of application: **09.10.2024**
(21) Application number: 22901796.7
(22) Date of filing: 30.11.2022
(51) Int. Cl.: C07K 16/28

(54) **SMO HUMAN ANTIBODY**

(30) Priority: 30.11.2021 KR 20210169089; 22.11.2022 KR 20220156920
(71) Applicant: BIOCOMPLETE INC., Seoul, 08285 (KR); Korea University Research and Business Foundation, Seoul 02841 (KR)
(72) Inventor: KIM, Bo Ram, Anyang-si Gyeonggi-do 13903 (KR); HUR, Minkyu, Gyeonggi-do 16819 (KR); LIM, Haet Nim, Seoul 03939 (KR); LEE, Jisun, Seoul 03472 (KR); OH, Sang Cheul, Seoul 06289 (KR); JUNG, Sang Taek, Seoul 06217 (KR)
(74) Representative: V.O.
(86) International application number: PCT/KR2022/019261
(87) International publication number: WO 2023/101438

(57) **Abstract**

The present disclosure relates to a novel human antibody or an antigen-binding fragment thereof that recognizes the SMO (smoothened) protein as an antigen and binds specifically thereto. The antibody of the present disclosure binds specifically to the SMO protein, which is directly involved in major cancer progression processes such as proliferation, migration, invasion and metastasis of cancer cells, and blocks the progression those processes. Therefore, it can be usefully used as a therapeutic agent for various cancers such as colon cancer, etc., resistant cancer, metastatic cancer, or recurrent cancer.

## Description

### [Technical Field]

The present disclosure relates to a monoclonal antibody or an antigen-binding fragment thereof that recognizes the SMO protein as an antigen and binds specifically thereto.

### [Background Art]

Cancer is the number one cause of death in Korea. Particularly, colon cancer has the highest incidence and mortality rates. Most recently, the incidence and mortality rates of colon cancer have been increasing rapidly in Korea. In Korea, many patients are diagnosed and treated at an early stage when surgery is possible through the early colon cancer screening program using colonoscopy. Nevertheless, one in four or five people has the cancer that has already spread to other organs at the time of diagnosis, and is diagnosed at stage 4 where surgery is not an option. Therapies or therapeutic agents for cancer have been developed in various fields, but usually, surgery, chemotherapy and radiation therapy are used in combination or alone depending on the stage of cancer.

Currently, the main goal of treatment for general cancer patients is to extend survival time and relieve symptoms. So, although the currently developed treatment methods may be superior in prolonging survival time or alleviating symptoms, they are still unsatisfactory in terms of the anticancer effect of completely curing the cancer.

Meanwhile, various targeted therapeutic agents have been developed, including bevacizumab (brand name: Avastin) and cetuximab (brand name: Erbitux). Specifically, bevacizumab is an anticancer drug that inhibits the growth and metastasis of cancer cells by identifying the interaction between cancer cells and VEGF (vascular endothelial growth factor) and blocking the VEGF pathway to inhibit angiogenesis. It is mainly used in combination with other anticancer drugs to enhance therapeutic effect. Since predictive indicators that can predict their effectiveness in advance have not been identified for the VEGF-targeted drugs such as bevacizumab, they are currently used without diagnosis such as testing of specific genes.

Additionally, cetuximab (brand name: Erbitux) is an anticancer drug that has the effect of inhibiting cancer growth by blocking the signaling system of cancer cells, and is a representative targeted therapeutic agent for colon cancer. It is an antibody against the epidermal growth factor receptor, which has the effect of inhibiting the growth of cancer cells by preventing cell division through the signals transmitted from growth factors.

In addition, various researches are being conducted actively around the world to develop effective methods and drugs not only for general cancer but also for cancers resistant to anticancer drugs or recurring cancer. However, most cancer therapies focus on reducing the size of cancer, and researches on cancers resistant to anticancer drugs or recurring cancer are still insufficient. In particular, what is important in determining the stage of cancer is not the size of the cancer but the degree to which the cancer has penetrated tissues. Therefore, there is a growing need to elucidate the biomolecules related to the symptoms of cancer such as cancer growth, metastasis, and resistant cancer and discover new biomarkers for targeted cancer therapy that can target and control both cancer growth and metastasis.

The matters described above as the background art are only for the purpose of improving the understanding of the background of the present disclosure, and should not be taken as an acknowledgment that they correspond to the prior art already known to those skilled in the art.

### [Disclosure]

### [Technical Problem]

The inventors of the present disclosure have identified that the SMO protein is related not only to general cancer but also to the growth and metastasis of anticancer drug-resistant cancer in the hedgehog (Hh) signaling pathway, and have made consistent efforts to discover a new anti-SMO human antibody capable of regulating the growth and metastasis cancer/resistant cancer by targeting the same. As a result, they have completed the present disclosure.

Therefore, the present disclosure is directed to providing a monoclonal antibody or an antigen-binding fragment thereof that recognizes the SMO (smoothened) protein as an antigen and binds specifically thereto.

The present disclosure is also directed to providing a nucleic acid molecule encoding the monoclonal antibody or an antigen-binding fragment thereof.

The present disclosure is also directed to providing a vector containing the above-described nucleic acid molecule.

The present disclosure is also directed to providing a host cell containing the vector.

The present disclosure is also directed to providing a pharmaceutical composition for preventing or treating cancer, which contains the above-described monoclonal antibody or an antigen-binding fragment thereof, a nucleic acid molecule encoding the monoclonal antibody or the antigen-binding fragment thereof, or a vector containing the nucleic acid molecule.

The present disclosure is also directed to providing a method for preventing or treating cancer, which includes a step of administering a therapeutically effective amount of the above-described monoclonal antibody or an antigen-binding fragment thereof, a nucleic acid molecule encoding the monoclonal antibody or the antigen-binding fragment thereof, or a vector containing the nucleic acid molecule to a subject in need thereof.

The present disclosure is also directed to providing a use of the above-described monoclonal antibody or an antigen-binding fragment thereof, a nucleic acid molecule encoding the monoclonal antibody or the antigen-binding fragment thereof, or a vector containing the nucleic acid molecule in therapy.

The present disclosure is also directed to providing a method for quantifying the SMO (smoothened) protein contained in a sample, which includes a step of treating the sample isolated from a subject with the above-described monoclonal antibody or an antigen-binding fragment thereof.

The present disclosure is also directed to providing a method for providing information for diagnosis of a disease caused by the overexpression of the SMO (smoothened) protein.

The present disclosure is also directed to providing a SMO (smoothened) protein quantification kit containing the above-described monoclonal antibody or an antigen-binding fragment thereof.

The present disclosure is also directed to providing a composition for diagnosing cancer, which contains the above-described monoclonal antibody or an antigen-binding fragment thereof.

Other purposes and advantages of the present disclosure become more apparent by the following detailed description, claims and drawings.

### [Technical Solution]

According to an aspect of the present disclosure, the present disclosure provides a monoclonal antibody or an antigen-binding fragment thereof that recognizes the SMO (smoothened) protein as an antigen and binds specifically thereto.

Hedgehog (Hh) is a protein that plays an important role in the embryonic development process. It acts on the formation of the dorsal and ventral axes during embryo pattern formation and the development of various organs including the nervous system and musculoskeletal system by regulating cell proliferation, migration and differentiation. In adults, hedgehog is involved in the maintenance of stem cells. Abnormal hyperactivity of hedgehog in adult tissues is known to cause various cancers such as basal-cell carcinoma, medullary brain cancer, etc. Accordingly, in adults, hedgehog signaling activity is maintained only in some specific tissues and is reduced in most somatic cells after the developmental process. On the other hand, it is known that excessive signaling activity caused by mutation in hedgehog signaling genes (Ptch, SMO, SUFU, Gli, etc.) in somatic cells induces carcinogenesis.

The hedgehog signaling pathway begins when sonic hedgehog binds to Patched1 (Ptch1), which is a 12-transmembrane receptor on the cell membrane. Ptch1 inhibits SMO, which is a 7-transmembrane Gli protein-binding receptor, but it loses its ability to inhibit SMO when sonic hedgehog binds to Ptch1. As a result, SMO is activated, and the transcription factor Gli is activated by the activated SMO, thereby activating the expression of hedgehog target genes. In other words, hedgehog, which acts as a ligand, binds to the Ptch1 receptor present in the cell membrane, thereby promoting the translocation of SMO, which is another GPCR-like transmembrane protein whose activity is suppressed by Phch1, to the primary cilium, and promoting the activity of the Gli transcription factor, which is responsible for the final stage of hedgehog signaling.

SMO regulates the activity of the Gli transcription factor by regulating SUFU, which inhibits the movement of the Gli transcription factor that has been translocated to the primary cilium by hedgehog to the nucleus in the downstream signaling stage, or increasing the stability of the Gli protein within the primary cilium and inducing the conversion of Gli from an inactive state to a transcriptionally active state, leading to increased expression of the genes affected by hedgehog by the Gli transcription factor and thereby allowing cell migration, proliferation and differentiation processes.

The inventors of the present disclosure made efforts to discover a new human antibody that can regulate cancer and, as a result, have completed the present disclosure by identifying a human antibody that binds specifically to the SMO protein, which is associated with various cancers in the hedgehog signaling pathway (Sonic (SHH)/Patched/(PTCH1)/Smoothened (SMO)).

In the present disclosure, the SMO protein can be represented by SEQ ID NO: 40, and its sequence information can be found in GenBank.

SMO (smoothened) is a 7-transmembrane type Gli protein-coupled receptor and is known to regulate the activity of the Gli transcription factor, which is responsible for the final stage of the hedgehog signaling pathway. However, so far, in order to suppress cancer caused by the hyperactivity of hedgehog signaling, new compounds have been developed which target the activity of Ptch1 or the hedgehog protein in the hedgehog signaling pathway, or inhibit the inhibitory activity of SMO. In this case, there is a problem that it is difficult to completely inhibit the hedgehog signaling pathway, and application to cancer cells resistant to anticancer drugs owing to SMO mutations is difficult. Therefore, as mentioned above, the development of an SMO inhibitor that exhibits an effective therapeutic effect against various cancers (general cancer, resistant cancer, recurrent cancer, etc.) is urgently needed.

It is known that abnormal hyperactivity (overexpression) of hedgehog in adult tissues causes various cancers such as basal-cell carcinoma, medullary brain cancer, etc. There are three types of mammalian hedgehog: Sonic hedgehog, Indian hedgehog and Desert hedgehog. The most studied one among them is the Sonic hedgehog (SHH) signaling pathway, which can be expressed throughout the body.

It was confirmed that the SMO protein is highly activated in cancer cells, and the high expression or activation of the SMO protein is associated with low survival rate of cancer patients or cancer cells.

The inventors of the present disclosure have identified that high expression of the SMO protein is associated with low survival rate of colon cancer patients, and that the expression of the SMO protein is increased in colon cancer cells as compared to normal cells. In addition, it was confirmed that the expression of the SMO and GLI1 proteins among hedgehog signaling components is increased in DLD-1, HCT15, HCT116, HT29, WIDR, SW48, SNUC2A, colo205, SNU283, SW480, SW620, HCT8, SNUC1 and LS174T colon cancer cell lines. In other words, it can be seen that the Gli protein, which is responsible for the final stage of hedgehog signaling, is regulated due to the activation of the SMO protein.

As used herein, the term "antibody" may refer to any type of immunoglobulin molecules (e.g., IgG, IgE, IgM, IgD, IgA or IgY), or any subtype of the antibodies (e.g., IgG1, IgG2, IgG3 and IgG4 in human; andlgG1, IgG2a, IgG2b and IgG3 in mice). Immunoglobulins (e.g., IgG1) may exist in various allotypes, and the term "antibody" used herein includes generally known isotypes and allotypes. In addition, the term "antibody" used herein may refer to IgG1, IgG2, IgG3, or IgG4, or a hybrid thereof (e.g., a hybrid of IgG2 and IgG4).

As used herein, the term "monoclonal antibody" refers to an antibody that exhibits single binding specificity and affinity for a particular epitope.

The monoclonal antibodies of the present disclosure may be produced, for example, by the hybridoma method first described in [Kohler et al., Nature 256, 495 (1975)], or may be produced by the recombinant DNA method. Monoclonal antibodies may also be isolated from phage antibody libraries using the technologies described, for example, in [Clackson et al., Nature 352, 624-628 (1991)] and [Marks et al., J. Mol. Biol. 222, 581-597 (1991)]. Monoclonal antibodies may be obtained from any suitable source. The monoclonal antibodies of the present disclosure may be acquired from hybridomas prepared from the cells expressing the SMO antigen or the cells obtained from mice immunized with the antigen of interest in the form of a nucleic acid encoding the SMO antigen. The monoclonal antibodies can also be obtained from the hybridomas derived from antibody-expressing cells of immunized human or non-human mammals, such as rats, dogs, primates, etc.

In this specification, the monoclonal antibody is used to include its fragments, and the fragment specifically refers to an antigen-binding fragment. The fragment can be prepared using various methods known in the art. For example, Fab and F(ab')₂ fragments can be prepared through proteolytic cleavage of immunoglobulin molecules using enzymes such as papain (for the Fab fragment) or pepsin (for the F(ab')₂ fragment).

As used herein, the term "fragment" may refer to Fab, Fab', F(ab')₂, Fv, scFv (single-chain Fv), or sdAb containing the V_{H} or V_{L} domain of a monomer. These fragments are well known in the art.

The monoclonal antibody of the present disclosure or a fragment thereof includes an antigen-binding protein that binds to the SMO protein represented by SEQ ID NO: 40, and it inhibits the physiological effects associated with cancer growth and metastasis mediated by SMO. Specifically, in cancer, the expression of the Gli protein, which is responsible for the final stage of hedgehog signaling, increases due to the activation of the hedgehog signaling mechanism, and it is involved in the expression of SMO by hedgehog during this process. The activation or expression of the SMO protein increases the Gli protein through the hedgehog signaling pathway and leads to cancer. In other words, the SMO protein increased or activated by the hedgehog signaling mechanism affects the growth and development of cancer, and the expression or activation of the SMO protein may be usefully suppressed by treatment with the monoclonal antibody containing the above-mentioned antigen-binding protein or a fragment thereof.

In the present disclosure, "inhibition of growth" refers to any measurable reduction in cell growth (e.g. reduction of cell growth by about 10%, 20%, 30%, 40%, 50%, 60%, 70%, 80%, 90% or 100%) upon contact with the monoclonal antibody of the present disclosure or a fragment thereof as compared to the same cells not contacted with the monoclonal antibody or the fragment thereof, and the reduction of cell growth can occur by various mechanisms.

The antigen-binding protein contain one or more (e.g., 1, 2, 3, 4, 5 or 6) CDRs. The antigen-binding protein refers to a polypeptide that binds to SMO as an antigen and includes one or more complementarity-determining region (CDR) described herein. In the antigen-binding protein, the CDRs are oriented such that an appropriate antigen-binding property is achieved. The antigen-binding protein provided herein can inhibit, block, reduce or modulate the expression or activation of SMO. That is to say, the occurrence, growth, and metastasis of cancer mediated by the hedgehog signaling pathway can be suppressed by inhibiting SMO present in a subject.

In addition, it is known that the dysregulation of the hedgehog signaling pathway contributes to the development of cancer resistant to chemotherapy and radiotherapy, or recurrent or metastatic cancer. Although the underlying mechanism of the resistance has not been elucidated clearly, it was confirmed that when the SMO antibody of the present disclosure is administered to cancer cells resistant to existing anticancer drugs, the growth of the cancer cells is inhibited significantly. That is to say, it can be seen that the monoclonal antibody of the present disclosure or a fragment thereof has excellent therapeutic effect not only on general cancer, but also on resistant cancer, recurrent cancer and metastatic cancer.

A V_{H} domain or one or more CDR may be linked to a constant domain to form a heavy chain. Additionally, a V_{L} domain or one or more CDR may be linked to a constant domain to form a light chain. A full-length heavy chain and a full-length light chain combine to make up a full-length antibody.

The variable region of an immunoglobulin chain exhibits the same general structure of relatively conserved framework regions (FRs) joined by three hypervariable regions (more often called "complementarity-determining regions" or CDRs). The CDRs from the two chains of each of the above-mentioned heavy/light chain pairs are usually aligned by the framework regions, forming a structure that binds specifically to a specific epitope on the target protein (e.g., SMO). From N-terminal to C-terminal, both naturally occurring light and heavy chain variable regions correspond generally to these elements in the following order: FR1, CDR1, FR2, CDR2, FR3, CDR3 and FR4. A numbering system has been devised to assign numbers to the amino acids occupying positions within each of these domains. The numbering system can be found in the literature [Kabat Sequences of Proteins of Immunological Interest (1987 and 1991, NIH, Bethesda, MD)], [Chothia & Lesk, 1987, J. Mol. Biol. 196: 901-917] or [Chothia et al., 1989, Nature 342: 878-883].

A variety of heavy and light chain variable regions may be provided by the present disclosure, each of which can be attached to the heavy and light chain constant regions to form complete antibody heavy and light chains, respectively, as described above. Additionally, the formed individual heavy and light chain sequences can be combined to form a complete antibody structure.

According to a specific exemplary embodiment of the present disclosure, the antibody is a human antibody.

According to a specific exemplary embodiment of the present disclosure, the monoclonal antibody or an antigen-binding fragment thereof includes the following CDR regions:
(a) CDRH1 of SEQ ID NO: 1;
(b) CDRH2 of SEQ ID NO: 2;
(c) CDRH3 of SEQ ID NO: 3, or CDRH3 containing an amino acid in which the amino acid at a position selected from a group consisting of the 6th, 7th, 8th, 11th, 12th and 18th positions in SEQ ID NO: 3 is substituted with another amino acid;
(d) CDRL1 of SEQ ID NO: 4;
(e) CDRL2 of SEQ ID NO: 5; and
(f) CDRL3 of SEQ ID NO: 6, or CDRL3 containing an amino acid in which the amino acid at the 6th position in SEQ ID NO: 6 is substituted with another amino acid.

The 6th amino acid of SEQ ID NO: 3 may be substituted from Y (Tyr) to H (His).

The 7th amino acid of SEQ ID NO: 3 may be substituted from Y (Tyr) to L (Leu).

The 8th amino acid of SEQ ID NO: 3 may be substituted from G (Gly) to D (Asp).

The 11th amino acid of SEQ ID NO: 3 may be substituted from T (Thr) to N (Asn).

The 12th amino acid of SEQ ID NO: 3 may be substituted from Y (Tyr) to F (Phe).

The 18th amino acid of SEQ ID NO: 3 may be substituted from F (Phe) to V (Val).

The 6th amino acid of SEQ ID NO: 6 may be substituted from S (Ser) to P (Pro).

The CDRH3 may be selected from a group consisting of the amino acid sequences of SEQ ID NO: 3, SEQ ID NO: 7, SEQ ID NO: 8, SEQ ID NO: 9, SEQ ID NO: 10, SEQ ID NO: 11 and SEQ ID NO: 12.

The CDRL3 may be selected from a group consisting of the amino acid sequences of SEQ ID NO: 6 and SEQ ID NO: 13.

According to a specific exemplary embodiment of the present disclosure, the monoclonal antibody or an antigen-binding fragment thereof may include CDRH1 of SEQ ID NO: 1; CDRH2 of SEQ ID NO: 2; CDRH3 having an amino acid sequence represented by the following General Formula 1; CDRL1 of SEQ ID NO: 4; CDRL2 of SEQ ID NO: 5; and CDRL3 having an amino acid sequence represented by the following General Formula 2:

General Formula 1 A-R-R-A-H-**X1**-**X2**-**X3**-G-S-**X4**-**X5**-N-P-S-W-Y-**X6**-D-L;

General Formula 2 Q-Q-G-Y-S-**X7**-P-R-T;

In General Formula 1, X1 is Y or H, X2 is Y or L, X3 is G or D, X4 is T or N, X5 is Y or F, X6 is F or V, and, in General Formula 2, X7 is S or P.

According to a specific exemplary embodiment of the present disclosure, the monoclonal antibody or an antigen-binding fragment thereof includes CDRH1 of SEQ ID NO: 14; CDRH2 of SEQ ID NO: 15; CDRH3 of SEQ ID NO: 16; CDRL1 of SEQ ID NO: 17; CDRL2 of SEQ ID NO: 18; and CDRL3 of SEQ ID NO: 19.

According to another aspect of the present disclosure, the present disclosure provides a nucleic acid molecule encoding the monoclonal antibody or an antigen-binding fragment thereof, a vector containing the nucleic acid molecule, or a host cell containing the vector.

The nucleic acid molecule of the present disclosure may be an isolated or recombinant nucleic acid molecule and includes single- and double-stranded forms of DNA and RNA as well as corresponding complementary sequences. The "isolated nucleic acid" means, in the case of a nucleic acid isolated from a naturally occurring source, a nucleic acid that has been separated from the surrounding genetic sequences present in the genome of the individual from which the nucleic acid has been isolated. In the case of nucleic acids synthesized enzymatically or chemically from a template, such as PCR products, cDNA molecules or oligonucleotides, the nucleic acids resulting from these procedures may be understood as isolated nucleic acid molecules. The isolated nucleic acid molecule refers to a nucleic acid molecule either in the form of an individual fragment or as a component of a larger nucleic acid construct. A nucleic acid is "operably linked" when it is placed into a functional relationship with another nucleic acid sequence. For example, the DNA of a presequence or a secretory leader is operably linked to the DNA of the polypeptide when expressed as a preprotein. A promoter or an enhancer affecting the transcription of the polypeptide sequence is operably linked to a coding sequence or a ribosome-binding site is operably linked to a coding sequence when it is arranged such that translation is promoted. In general, the term "operably linked" means that DNA sequences to be linked are located adjacent to each other. In the case of secretory leaders, the term "operably linked" means that the secretory leaders are present adjacent to each other in the same leading frame.

However, an enhancer needs not be contiguous. The linkage is performed by ligation at a convenient restriction enzyme site. In the case where such a site does not exist, a synthetic oligonucleotide adaptor or a linker is used according to a conventional method known in the art.

As used herein, the term "vector" refers to a carrier into which a nucleic acid sequence can be inserted for introduction into a cell where it can be replicated. A nucleic acid sequence may be exogenous or heterologous. Examples of the vector include, but are not limited to, a plasmid, a cosmid and a virus (e.g., bacteriophage, adeno-associated virus (AAV), etc.). Those skilled in the art can construct such vectors through standard recombinant techniques (Maniatis et al.,Molecular Cloning, A Laboratory Manual, Cold Spring Harbor Press, Cold Spring Harbor, N.Y., 1988; and Ausubel et al., In: Current Protocols in Molecular Biology, John, Wiley & Sons, Inc, N.Y., 1994, etc.).

As used herein, the term "expression vector" refers to a vector containing a nucleic acid sequence coding for at least part of a gene product to be transcribed. In some cases, the RNA molecule is then translated into a protein, a polypeptide or a peptide. The expression vector can contain a variety of regulatory sequences. In addition to a regulatory sequence that govern transcription and translation, the vector and the expression vector may contain nucleic acid sequences that serve other functions as well.

As used herein, the term "host cell" includes eukaryotic and prokaryotic cells, and refers to any transformable organism capable of replicating the vector or expressing the gene encoded by the vector. The host cell may be transfected or transformed by the vector, which refers to a process in which an exogenous nucleic acid molecule is transferred or introduced into the host cell.

The host cell of the present disclosure is not limited, but may be a eukaryotic cell, specifically an insect cell or a mammalian cell, more specifically Sf9 as an insect cell, or HEK293 cell, HeLa cell, ARPE-19 cells RPE-1 cell, HepG2 cell, Hep3B cell, Huh-7 cell, C8D1a cell, Neuro2A cell, CHO cell, MES13 cell, BHK-21 cell, COS7 cell, COP5 cell, A549 cell, MCF-7 cell, HC70 cell, HCC1428 cell, BT-549 cell, PC3 cell, LNCaP cell, Capan-1 cell, Panc-1 cell, MIA PaCa-2 cell, SW480 cell, HCT166 cell, LoVo cell, A172 cell, MKN-45 cell, MKN-74 cell , Kato-III cell, NCI-N87 cell, HT-144 cell, SK-MEL-2 cell, SH-SY5Y cell, C6 cell, HT-22 cell, PC-12 cell or NIH3T3 cell as a mammalian cell.

The host cell of the present disclosure is specifically an isolated host cell.

According to another aspect of the present disclosure, the present disclosure provides a pharmaceutical composition for preventing or treating cancer, which contains the above-described monoclonal antibody or an antigen-binding fragment thereof, a nucleic acid molecule encoding the monoclonal antibody or the antigen-binding fragment thereof, or a vector containing the nucleic acid molecule.

The pharmaceutical composition of the present disclosure may contain (a) the above-described monoclonal antibody or an antigen-binding fragment thereof, the above-described nucleic acid molecule, or a vector containing the nucleic acid molecule; and (b) a pharmaceutically acceptable carrier.

According to another aspect of the present disclosure, the present disclosure provides a method for preventing or treating cancer, which includes a step of administering a therapeutically effective amount of the above-described monoclonal antibody or the antigen-binding fragment thereof, the above-described nucleic acid molecule, or a vector containing the nucleic acid molecule to a subject in need thereof.

According to another aspect of the present disclosure, the present disclosure provides a therapeutic use of the monoclonal antibody or an antigen-binding fragment thereof, a nucleic acid molecule encoding the monoclonal antibody or an antigen-binding fragment thereof, or a vector containing the nucleic acid molecule.

According to a specific exemplary embodiment of the present disclosure, the therapeutic use is treatment of cancer.

The type of cancer to be prevent or treated in the present disclosure is not limited, and includes various cancers including leukemias such as acute lymphocytic leukemia, acute nonlymphocytic leukemia, chronic lymphocytic leukemia and chronic myelogenous leukemia, lymphomas such as Hodgkin's disease, non-Hodgkin's lymphoma, multiple myeloma, etc., childhood solid tumors such as brain tumor, glioblastoma, neuroblastoma, rhabdomyosarcoma, retinoblastoma, Wilms tumor, bone tumor, soft-tissue sarcoma, etc., and common solid tumors in adults such as lung cancer, breast cancer, prostate cancer, urinary cancers, uterine cancer, oral cancer, pancreatic cancer, melanoma and other skin cancers, stomach cancer, colon cancer, ovarian cancer, brain tumor, liver cancer, laryngeal cancer, thyroid cancer, esophageal cancer, testicular cancer, etc.

Additionally, the cancer may be advanced cancer, resistant cancer, recurrent cancer or metastatic cancer. In particular, the resistant cancer may be cancer that is resistant to cancer-treating drugs, and the cancer-treating drugs include nitrogen mustard, imatinib, oxaliplatin, rituximab, erlotinib, neratinib, lapatinib, gefitinib, vandetanib, nilotinib, semaxanib, bosutinib, axitinib, cediranib, lestaurtinib, trastuzumab, bortezomib, sunitinib, carboplatin, sorafenib, bevacizumab, cisplatin, cetuximab, *Viscum album,* asparaginase, tretinoin, hydroxycarbamide, dasatinib, estramustine, gemtuzumab ozogamicin, ibritumomab tiuxetan, heptaplatin, methyl aminolevulinic acid, amsacrine, alemtuzumab, procarbazine, alprostadil, holmium nitrate chitosan, gemcitabine, doxyfluridine, pemetrexed, tegafur, capecitabine, gimeracin, oteracil, azacitidine , methotrexate, uracil, cytarabine, fluorouracil, fludarabine, enocitabine, flutamide, decitabine, mercaptopurine, thioguanine, cladribine, carmofur, raltitrexed, docetaxel, paclitaxel, irinotecan , belotecan, topotecan, vinorelbine, etoposide, vincristine, vinblastine, teniposide, doxorubicin, idarubicin, epirubicin, mitoxantrone, mitomycin, bleomycin, daunorubicin, dactinomycin, pirarubicin, aclarubicin, pepromycin, temsirolimus, temozolomide, busulfan, ifosfamide, cyclophosphamide, melphalan, altretamine, dacarbazine, thiotepa, nimustine, chlorambucil, mitolactol, leucovorin, tretonin, exemestane, aminoglutethimide, anagrelide, Navelbine, fadrazole, tamoxifen, toremifene, testolactone, anastrozole , letrozole, borozole, bicalutamide, lomustine, vorinostat, entinostat, 5FU, carmustine, etc.

As used herein, the term "prevention" refers to suppressing the occurrence of a disorder or a disease in a subject who has not been diagnosed as having the disorder or disease but is likely to develop the disorder or disease.

As used herein, the term "treatment" refers to (a) suppressing the development of a disorder, a disease or a symptom; (b) alleviating a disorder, a disease or a symptom; or (c) eliminating a disorder, a disease or a symptom. When a nucleic acid molecule encoding the monoclonal antibody of the present disclosure or an antigen-binding fragment thereof or a vector containing the nucleic acid molecule is administered to a subject, the proliferation of cancer cells is inhibited as the activity or expression of the SMO protein is inhibited and, as a result, the related symptoms are eliminated or alleviated. Therefore, the nucleic acid molecule encoding the monoclonal antibody of the present disclosure or an antigen-binding fragment thereof or the vector containing the nucleic acid molecule may be included in a composition for treating cancer by itself, or may be administered together with another pharmacological ingredient to improve therapeutic responsiveness as a therapeutic adjuvant. Accordingly, in this specification, the term "treatment" or "therapeutic agent" includes the meaning of "aid of treatment" or "therapeutic adjuvant".

As used herein, the term "administration" or "administer" refers to administering a pharmaceutically effective amount of the pharmaceutical composition of the present disclosure or a therapeutically effective amount of the nucleic acid molecule encoding the monoclonal antibody or an antigen-binding fragment thereof or a vector containing the nucleic acid molecule directly to a subject so that the same amount is formed in the subject's body.

In the present disclosure, the term "therapeutically effective amount" refers to the amount of the composition in which the pharmacological ingredient in the composition is contained in an amount sufficient to provide a therapeutic or preventive effect to the individual to whom the pharmaceutical composition of the present disclosure is to be administered and, thus, is meant to include "prophylactically effective amount."

As used herein, the term "subject" refers to mammals including, without limitation, human, mouse, rat, guinea pig, dog, cat, horse, cow, pig, monkey, chimpanzee, baboon, rhesus monkey, etc. Specifically, the subject of the present disclosure is human.

The pharmaceutically acceptable carrier contained in the pharmaceutical composition of the present disclosure is one commonly used in preparation and includes lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, etc., although not being limited thereto. In addition to the above ingredients, the pharmaceutical composition of the present disclosure may further contain a lubricant, a wetting agent, a sweetener, a flavoring agent, an emulsifier, a suspending agent, a preservative, etc. Suitable pharmaceutically acceptable carriers and preparations are described in detail in Remington's Pharmaceutical Sciences (19th ed., 1995).

The antibody (including the nucleic acid encoding the same or the vector) or the pharmaceutical composition of the present disclosure can be administered orally or parenterally, specifically parenterally, for example, by intravenous injection, transdermal administration, subcutaneous injection, intramuscular injection, intravitreal injection, subretinal injection, suprachoroidal injection, eye drop administration, intracerebroventricular injection, intrathecal injection , intraamniotic injection, intraarterial injection, intraarticular injection, intracardiac injection, intracavernous injection, intracerebral injection, intracisternal injection, intracoronary injection, intracranial injection, intradural injection, epidural injection, intrahippocampal injection, intranasal injection, intraosseous injection, intrapleural injection, intraspinal injection, intrathoracic injection, intrathymic injection, intrauterine injection, intravaginal injection, intraventricular injection, intravesical injection, subconjunctival injection, intratumoral injection, topical injection, intraperitoneal injection, etc.

An appropriate dosage of the pharmaceutical composition of the present disclosure varies depending on factors such as preparation method, administration method, the age, body weight, sex and pathological condition of a patient, diet, administration time, administration route, excretion rate, and response sensitivity. An ordinarily skilled physician can easily determine and prescribe an effective dosage for the desired treatment or prevention. According to a specific exemplary embodiment of the present disclosure, a daily dosage of the pharmaceutical composition of the present disclosure is 0.0001-100 mg/kg.

The pharmaceutical composition of the present disclosure may be prepared as a unit-dose form using a pharmaceutically acceptable carrier and/or excipient or may be packaged in a multi-dose container according to a method that may be easily carried out by a person having common knowledge in the art to which the present disclosure belongs. A formulation may be in the form of a solution, a suspension or an emulsion in an oily or aqueous medium, or may be in the form of an extract, a powder, a granule, a tablet or a capsule, and may additionally contain a dispersant or a stabilizer. An appropriate dosage of the pharmaceutical composition of the present disclosure varies depending on factors such as preparation method, administration method, the age, body weight, sex and pathological condition of a patient, diet, administration time, administration route, excretion rate, and response sensitivity. An ordinarily skilled physician can easily determine and prescribe an effective dosage for the desired treatment or prevention.

The pharmaceutical composition of the present disclosure can be used for a stand-alone therapy, but can also be used in combination with another conventional chemotherapy or radiation therapy, and when such a combination therapy is performed, cancer may be treated more effectively. Chemotherapeutic agents that can be used together with the composition of the present disclosure include cisplatin, carboplatin, procarbazine, mechlorethamine, cyclophosphamide, ifosfamide, melphalan, chlorambucil, busulfan, nitrosourea, dactinomycin, daunorubicin, doxorubicin, bleomycin, plicamycin, mitomycin, etoposide, tamoxifen, Taxol, transplatinum, 5-fluorouracil, vincristine, vinblastine, methotrexate, etc. The radiation therapy that can be used together with the composition of the present disclosure includes X-ray irradiation, γ-ray irradiation, etc.

According to another aspect of the present disclosure, the present disclosure provides a method for quantifying the SMO protein contained in a sample, which includes a step of treating a sample isolated from a subject with the above-described monoclonal antibody or a fragment thereof.

Since the monoclonal antibody of the present disclosure or a fragment thereof binds specifically to the SMO protein, it can be used to accurately measure the expression level of the SMO protein contained in the sample. In addition, the expression level of various SMO proteins associated with cancer resistant to cancer-treating drugs, recurrent cancer, or metastatic cancer can also be measured.

According to another aspect, the present disclosure provides a method for providing information for diagnosis of a disease caused by overexpression of the SMO protein, which includes:
(a) a step of obtaining a sample isolated from a subject;
(b) a step of treating the sample with the above-described monoclonal antibody or an antigen-binding fragment thereof; and
(c) a step of checking whether the expression level of SMO contained in the subject's sample is higher than the expression level of SMO contained in a normal group sample.

The SMO protein is overexpressed in various cancers such as colon cancer, cancers resistant to cancer-treating drugs, metastatic cancer, and recurrent cancer, and is directly involved in major cancer progression processes such as proliferation, migration, invasion, metastasis, etc. of cancer cells. Therefore, information for diagnosis of a disease caused by overexpression of the SMO protein may be provided by comparing the expression level of the SMO protein with that of a normal person.

According to a specific exemplary embodiment of the present disclosure, the disease caused by overexpression of the SMO protein is cancer.

According to another aspect of the present disclosure, the present disclosure provides a SMO (smoothened) protein quantification kit containing the above-described monoclonal antibody or an antigen-binding fragment thereof.

The quantification kit of the present disclosure can quantify the amount of the SMO protein by analyzing the antigen for the antibody through an antigen-antibody binding reaction, and the antigen-antibody binding reaction may be analyzed by a common method selected from a group consisting of ELISA (enzyme-linked immunosorbent assay), RIA (radioimmnoassay), sandwich assay, western blot on polyacrylamide gel, immunoblot assay, and immunohistochemical staining, although not being limited thereto.

As a support for the antigen-antibody binding reaction, one selected from a group consisting of a nitrocellulose membrane, a PVDF membrane, a well plate synthesized from polyvinyl resin or polystyrene resin, and a slide glass made of glass may be used, although not being limited thereto.

It is preferred that a secondary antibody is labeled with a common color developer for a chromogenic reaction. One or more fluorophore or dye selected from a group consisting of HRP (horseradish peroxidase), alkaline phosphatase, colloidal gold, or FITC (poly-L-lysine-fluorescein isothiocyanate), RITC (rhodamine B isothiocyanate), etc. may be used as the label. It is preferred that a substrate that induces color development is determined depending on the label that participates in the chromogenic reaction. Specifically, any one selected from a group consisting of TMB (3,3',5,5'-tetramethyl bezidine), ABTS [2,2'-azino-bis(3-ethylbenzothiazoline-6-sulfonic acid)] and OPD (O-phenylenediamine) may be used, although not being limited thereto.

According to another aspect of the present disclosure, the present disclosure provides a composition for diagnosing cancer, which contains the above-described monoclonal antibody or an antigen-binding fragment thereof.

Since the antibody or the antigen-binding fragment thereof used in the present disclosure and the cancer targeted thereby have already been described in detail, their description will be omitted to avoid excessive redundancy.

In this specification, the term "diagnosis" includes determination of the susceptibility of an individual (subject) to a specific disease, determination of whether an individual currently has a specific disease, and determination of the prognosis of an individual suffering from a specific disease.

Since the SMO protein is overexpressed in various cancer tissues and is directly involved in major cancer progression processes such as proliferation, migration, invasion, metastasis, etc. of cancer cells, it can be used as a diagnostic marker for cancer. The antibody of the present disclosure, which binds specifically to SMO, can accurately determine the presence of a tumor in a biological sample.

As used herein, the term "diagnostic composition" refers to an integrated mixture or device containing the antibody of the present disclosure as a means of determining whether cancer has developed in a subject or measuring the expression level of the SMO protein, and may also be referred to as a "diagnostic kit".

As used herein, the term "sample" or "biological sample" includes a tissue, a cell, whole blood, serum, plasma, saliva, urine, lymph, spinal fluid, a tissue autopsy sample (brain, skin, lymph node, spinal cord, etc.), a cell culture supernatant, a disrupted eukaryotic cell, and a bacterial expression system, although not being limited thereto.

### [Advantageous Effects]

The features and advantages of the present disclosure are summarized as follows:
(i) The present disclosure provides a novel human antibody that recognizes the SMO (smoothened) protein as an antigen and binds specifically thereto, or an antigen-binding fragment thereof.
(ii) The antibody of the present disclosure binds specifically to the SMO protein, which is directly involved in major cancer progression processes such as proliferation, migration, invasion and metastasis of cancer cells, and blocks the progression those processes. Therefore, it can be usefully used as a therapeutic agent for various cancers such as colon cancer, etc., resistant cancer, metastatic cancer, or recurrent cancer.

### [Brief Description of Drawings]

FIG. 1 shows the expression vector for animal cells of the prepared dimeric and tetrameric SMO CRD region antigen proteins and an SDS-PAGE gel photograph after expression and purification in animal cells.
FIG. 2 is a schematic diagram of antibody screening by flow cytometry.
FIG. 3 shows a result of analyzing the SMO CRD binding affinity of SMO scFv antibody variants by flow cytometry.
FIG. 4 shows a result of analyzing the amino acid sequence of scFv human antibody variants showing high binding affinity to the SMO CRD discovered through search.
FIG. 5 shows animal cell expression vectors for three types of antibodies and an SDS-PAGE gel image obtained after expression and in animal cells and purification.
FIG. 6 shows a result of ELISA performed to confirm the binding ability of three types of antibodies to SMO.
FIG. 7 shows a result of flow cytometry performed to confirm the binding ability of three types of antibodies to SMO.
FIG. 8 shows a result of GLI luciferase activity measurement using NIH3T3 GLI cells to confirm the efficacy of three types of antibodies against SMO.
FIG. 9 shows the FACS data of scFv human antibody variants with increased binding affinity to the SMO CRD discovered through search.
FIG. 10 shows a result of confirming the improvement of the SMO CRD binding ability of SMO-binding scFv antibody variants through flow cytometry.
FIG. 11 shows SDS-PAGE gel images obtained after expression of an antibody in animal cells and purification.
FIG. 12 shows a result of ELISA performed to confirm the binding affinity of an antibody with improved binding affinity to SMO.
FIG. 13 shows a result of flow cytometry performed to confirm the binding affinity of an antibody with improved binding affinity to SMO.
FIG. 14 shows a result of measuring GLI luciferase activity increased by SHH in NIH3T3 GLI cells to confirm the efficacy of four antibodies against SMO.
FIG. 15 shows a result of measuring GLI luciferase activity increased by hydroxysterol 20(s) in NIH3T3 GLI cells to confirm the efficacy of four antibodies against SMO.
FIG. 16 shows a result of transwell migration assay using SKOV3 cancer cells to confirm the ability of an SMO antibody to inhibit cancer migration.

### [Mode for Invention]

Hereafter, the present disclosure will be described in more detail through examples. These examples are intended solely to explain the present disclosure in more detail, and it will be obvious to those having common knowledge in the art that the scope of the present disclosure is not limited by the examples.

### Examples

### <Example 1> Antigen production using the CRD (Cysteine rich domain) region of the extracellular region of SMO

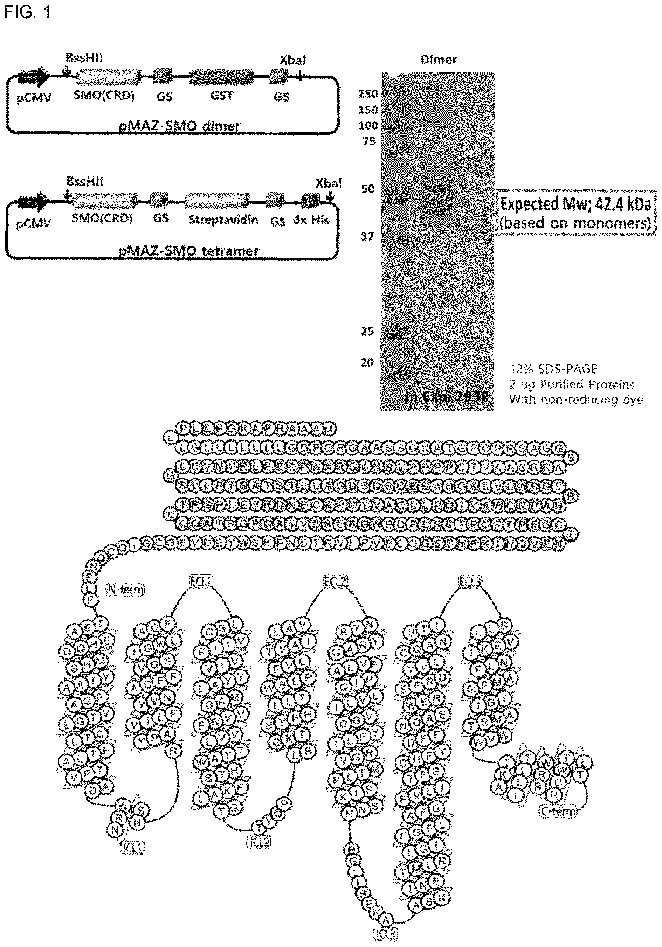

The CRD (cystine-rich domain) region of SMO was prepared as an antigen to discover IgG that binds to SMO. In addition, in order to prepare antigens more suitable for antibody screening by flow cytometry, an expression vector for animal cells to prepare antigens in a dimeric form fused with a GST (glutathione S-transferase) tag and a tetrameric form fused with a streptavidin tag were constructed (FIG. 1). Afterwards, it was transfected into Expi293F animal cells. One day before the transfection, the Expi293F cells were subcultured to a density of 2×10⁶ cells/mL in 300 mL and transfected the next day using polyethylenimine (PEI, Polyscience, 23966). First, each gene was mixed in 30 mL of a Freestyle 293 expression culture medium (GIBCO, 12338-018), and then mixed with PEI:variant gene at a ratio of 4:1. After leaving at room temperature for 20 minutes, followed by mixing with the cells subcultured the previous day, the cells were cultured in a shaking CO₂ incubator at 37 °C, 125 rpm and 8% CO₂ for 7 days. The cells were centrifuged and only the supernatant was collected. Afterwards, equilibrium was achieved using 25x PBS. The culture was filtered with a 0.2-µm bottle top filter (Merck Millipore). After adding 1 mL of GSH resin and Ni-NTA resin to the filtered culture and stirring for 16 hours at 4 °C, the resin was recovered and washed with 5 mL of PBS. Then, the culture was eluted with 4 mL of 50 mM Tris-HCl and 10 mM reduced glutathione (Sigma) with 250 mM imidazole at pH 8.0. After exchanging buffer to PBS using Centrifugal Filter Units 3K (Merck Millipore), the size and purity of the two purified antigen proteins were analyzed by SDS-PAGE (FIG. 1).

### <Example 2> Human antibody screening using an Alexa 488-labeled dimeric SMO CRD region and high-throughput screening system by flow cytometry

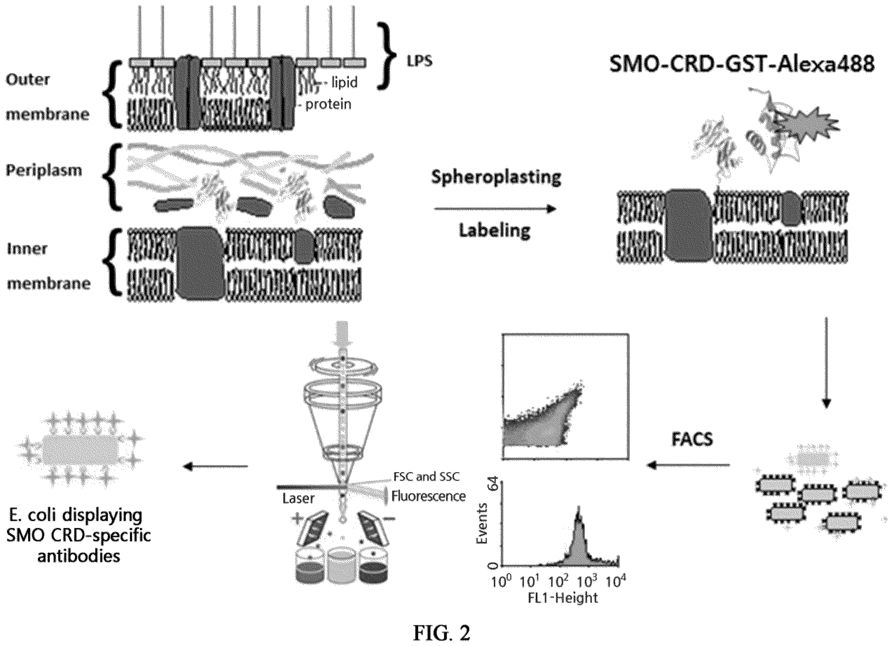

Among the previously prepared antigen proteins, the GST (glutathione S-transferase)-fused antigen protein was labeled with the Alexa 488 fluorophore for use in screening using by flow cytometry. The inventors of the present disclosure conducted screening for human antibodies binding to the SMO CRD region using a bacterially displayed human scFv antibody library constructed in the previous research. 1 mL of library cells were cultured in 25 mL of a TB medium containing 2% glucose and 40 µg/mL chloramphenicol at 37 °C and 250 rpm for 4 hours. The cultured cells were cultured in 100 mL of a TB medium containing 40 µg/mL chloramphenicol at a ratio of 1:100 to OD₆₀₀ = 0.5 and then cooled at 25°C and 250 rpm for 20 minutes. Then, after adding 1 mM IPTG, the cells were incubated at 25°C and 250 rpm for 25 minutes and *E*. *coli* overexpressing the protein was harvested based on OD₆₀₀ = 8 by induction at 25°C and 250 rpm for 5 hours. To transform the cells into spheroplasts suitable for screening by flow cytometry, the cells were washed twice by resuspension using 1 mL of 10 Mm Tris-HCl (pH 8.0) to remove the residual medium, and the outer cell membrane was removed by subjecting the cells to osmotic shock through rotation in a solution of 1 mL of STE [0.5 M sucrose, 10 Mm Tris-HCl, 10 Mm EDTA (pH 8.0)] at 37°C for 30 minutes. A mixture of 1 mL of solution A and 20 µL of 50 mg/mL lysozyme solution was rotated at 37°C for 15 minutes to remove the peptidoglycan layer. After washing with 1 mL of PBS, 300 µL of the solution was mixed with 700 µL of PBS and 200 nM SMO-CRD-GST-Alexa 488 probe (based on monomers) was added and rotated at room temperature for 1 hour to label the spheroplasts with the fluorescent probe. The clones exhibiting strong fluorescence due to increased antigen-binding ability were recovered by flow cytometry (FIG. 2). From the recovered clones, scFv genes were amplified by PCR, and the process of sorting spheroplasts exhibiting high affinity to the SMO CRD region through culture, expression induction, spheroplasting for removing the outer cell membrane and peptidoglycan layer, antigen labeling, and selective gating by flow cytometry was repeated. After the sorting process by flow cytometry and a resorting process to increase sorting purity, followed by an enrichment process, a process of securing the sorted scFv variant genes through PCR amplification, a subcloning process, and a transformation process, the next round of sorting process was performed to enrich the desired clones. Through repeated rounds, the scFv variant clones with excellent binding affinity to the SMO CRD region were obtained.

### <Example 3> Analysis of binding affinity to the SMO CRD region of selected antibody variants using flow cytometry

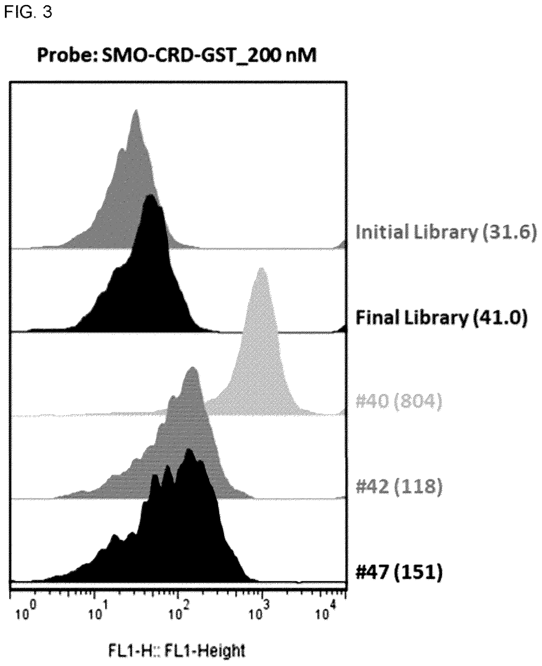

To confirm the binding affinity of scFv antibody variants to the SMO CRD region, binding affinity analysis was performed using flow cytometry. For this purpose, an initial library was constructed using the spheroplasts according to the previously used method. The SMO CRD-GST-Alexa 488 antigen used for the screening was bound to the prepared spheroplasts at a concentration of 200 nM and incubated for 1 hour at room temperature. After washing twice with PBS to remove non-specific binding, the binding ability to the SMO CRD region was analyzed by flow cytometry. As a result of the analysis, three scFv variants (FIG. 3) showed significantly increased fluorescence signals as compared to the control initial library, and through this, the scFv antibody variants that bind to the SMO CRD region were identified.

### <Example 4> Confirmation of gene sequences of SMO-binding antibody variants

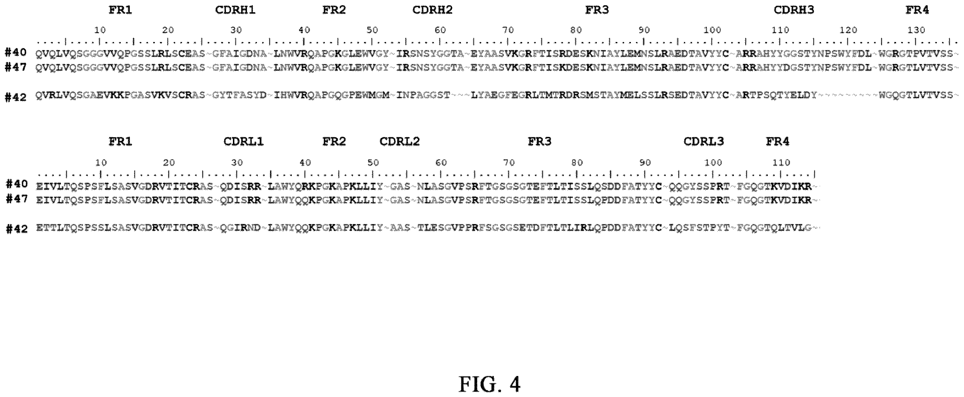

To confirm the gene sequences of the obtained scFv variant clones, DNA base sequences were analyzed by Sanger sequencing. Through the analysis, the amino acid sequences of the three scFv antibodies having binding ability to SMO were confirmed (FIG. 4).

### <Example 5> Production and purification of three scFv antibody variants

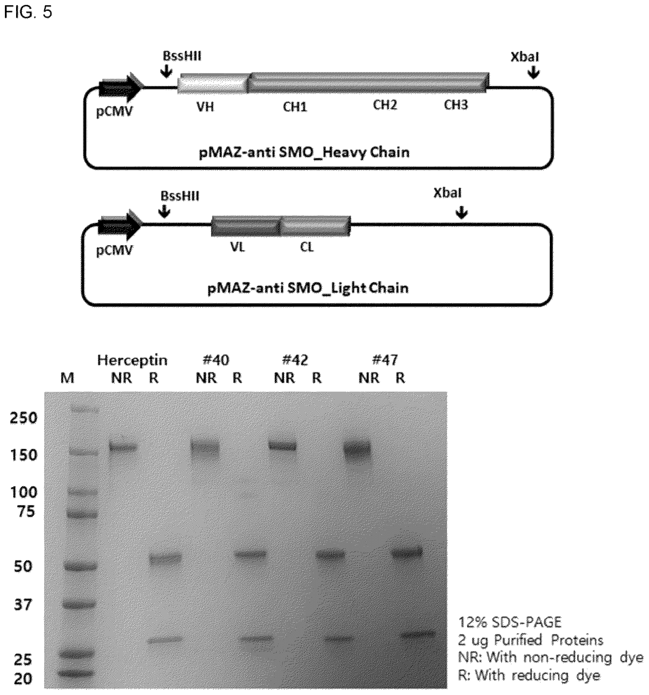

In order to confirm whether the three scFv antibody variants screened by the previous experiment have binding ability to SMO even in the form of IgG, heavy-chain and light-chain expression vectors for the three types of scFv were constructed, respectively. Then, they were transfected into Expi293F animal cells. One day before the transfection, the Expi293F cells were subcultured to a density of 2×10⁶ cells/mL and transfected the next day using polyethylenimine (PEI, Polyscience, 23966). First, the variants were mixed in 100 mL of a Freestyle 293 expression culture medium (GIBCO, 12338-018), and then mixed with PEI:variant gene at a ratio of 4:1. After leaving at room temperature for 20 minutes, followed by mixing with the cells subcultured the previous day, the cells were cultured in a shaking CO₂ incubator at 37°C, 125 rpm and 8% CO₂ for 7 days. The cells were centrifuged and only the supernatant was collected. Afterwards, equilibrium was achieved using 25x PBS. The culture was filtered with a 0.2-µm bottle top filter (Merck Millipore). 500 µL of protein A resin was added to the filtered culture medium, stirred at 4°C for 16 hours, flown through a column to recover the resin, washed with 5 mL of PBS, eluted with 3 mL of 100 mM glycine pH 2.7 buffer, and then neutralized using 1 M Tris-HCl pH 8.0. After exchanging the buffer with PBS using Centrifugal Filter Units 3K (Merck Millipore), the size and purity of each of the three purified antibodies were analyzed by SDS-PAGE (FIG. 5).

### <Example 6> Analysis of binding affinity of SMO-binding antibodies to SMO CRD

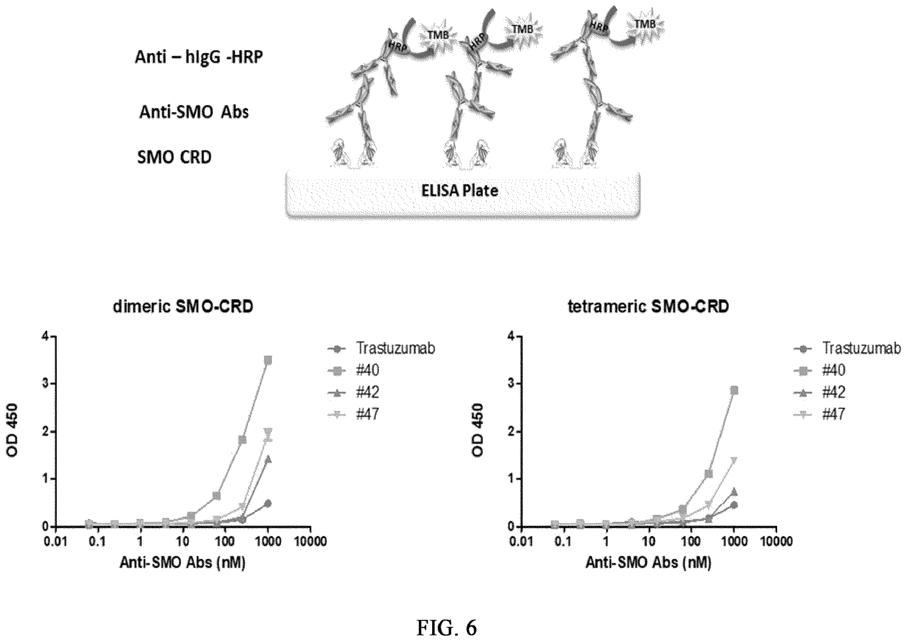

ELISA experiment was performed to measure the binding affinity of the antibodies to SMO. 50 µL of dimeric SMO CRD fused with a GST tag and tetrameric SMO CRD fused with a streptavidin tag at 4 µg/mL in 0.05 M Na₂CO₃ pH 9.6 were placed in different Flat Bottom Polystyrene High Bind 96-well microplates (Costar) at 4°C for 16 hours and then blocked with 100 µL of 4% skim milk (GenomicBase) (in 0.05% PBST pH 6.0/pH 7.4) for 2 hours at room temperature. After washing four times with 180 µL of 0.05% PBST, 50 µL of the three antibodies serially diluted with 1% skim milk (in 0.05% PBST) and trastuzumab, as a control group, were dispensed into each well and reacted at room temperature for 1 hour. After the washing process, antibody reaction was performed for 1 hour at room temperature using 50 µL of anti-Human HRP conjugate (Jackson Immunoresearch), followed by washing. 50 µL of 1-Step Ultra TMB-ELISA Substrate solution (Thermo Fisher Scientific) was added to develop color, and the reaction was terminated by adding 50 µL of 2 M H₂SO₄, followed by analysis using an Epoch microplate spectrophotometer (BioTek) (FIG. 6).

### <Example 7> Confirmation of binding ability of SMO-binding antibodies to SMO in HCT116 cells

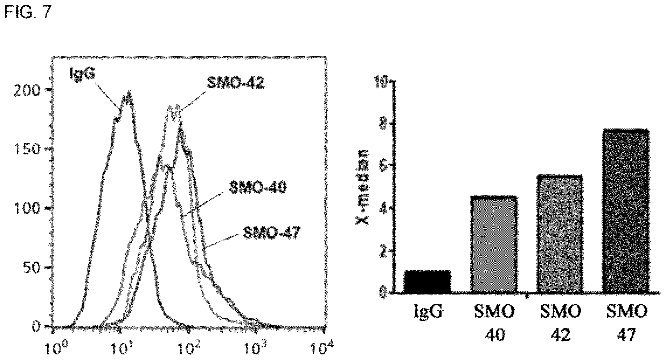

The binding affinity of the SMO-binding antibodies in HCT116 colon cancer cells was analyzed by flow cytometry. After blocking the HCT116 cells with BSA for 30 minutes, 10 µg of the SMO-binding antibody was bound and incubated for 1 hour at room temperature. One hour later, the cells were washed with PBS, reacted with 2 µL of Alexa 594 secondary antibody, and then incubated for 1 hour at room temperature. One hour later, the cells were washed with PBS to remove non-specific binding, and the binding ability of the SMO-binding antibodies was analyzed by flow cytometry. As a result of the analysis, it was confirmed that the binding affinity of the three scFv variants to SMO in the HCT116 cells increases in the order of 47, 42 and 40 (FIG. 7).

### <Example 8> Confirmation of inhibition of hedgehog signaling by SMO-binding antibodies

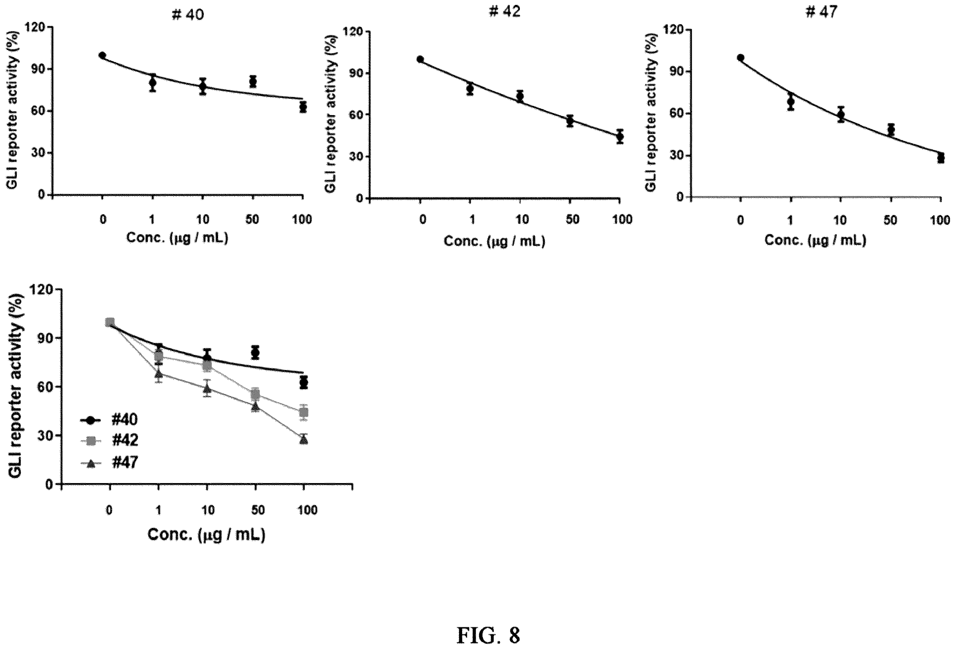

To determine whether the antibodies of the present disclosure inhibit hedgehog signaling, experiment was performed as follows. In hedgehog signaling, signals are transmitted to PTCH and SMO receptors by hedgehog ligands, and the GLI protein, which is a transcription factor in the final stage, regulates the expression of various genes. To confirm this signaling, cell-based assay was performed in NIH3T3 cells containing a reporter plasmid with luciferase bound to the GLI protein-binding site. The NIH3T3 GLI cells were seeded on a 96-well plate at a density of 2.5×10⁴ cells per well, and treated with the SMO-binding antibody at concentrations of 1, 10, 50 and 100 µg the next day. 2 hours later, the cells were treated with the ligands oxysterol 20(s) and oxysterol 25 at 7.5 µM. After treating with the antibody for 24 hours, luciferase activity was measured using a Promega kit. As a result of the measurement, it was confirmed that the ability of inhibiting GLI transcriptional activity of the three scFv variants in the NIH3T3 GLI cells increased in the same order as 47, 42, and 40 as the binding affinity determined by flow cytometry (FIG. 8).

### <Example 9> Screening of human antibodies with improved binding affinity to SMO CRD region using high-throughput screening system by flow cytometry

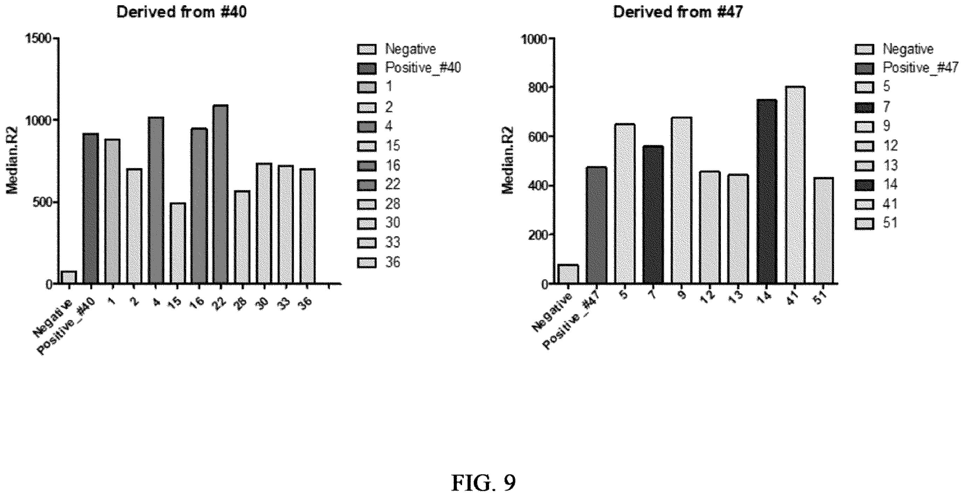

Experiment was designed to increase the binding affinity of the three types of antibodies with confirmed binding affinity to SMO CRD, as parent antibodies, to SMO. For this purpose, a CDRH3 NNK library was constructed by substituting the amino acids of CDR (complementarity-determining domain) 3 in the heavy chains of the antibodies #40, #42 and #47 one by one. In addition, screening of human antibodies binding to the SMO CRD region was conducted using the SMO-CRD-GST-Alexa 488 probe, which was the antigen used to discover antibodies, and the error-prone library of bacterially displayed human scFv antibodies. 1 mL of the library cells were cultured in 25 mL of a TB medium containing 2% glucose and 40 µg/mL chloramphenicol at 37 °C and 250 rpm for 4 hours. The cultured cells were cultured in 100 mL of a TB medium containing 40 µg/mL chloramphenicol at a ratio of 1:100 to OD₆₀₀ = 0.5 and then cooled at 25°C and 250 rpm for 20 minutes. Then, after adding 1 mM IPTG, the cells were incubated at 25°C and 250 rpm for 25 minutes and E. *coli* overexpressing the protein was harvested based on OD₆₀₀ = 8 by induction at 25°C and 250 rpm for 5 hours. To transform the cells into spheroplasts suitable for screening by flow cytometry, the cells were washed twice by resuspension using 1 mL of 10 Mm Tris-HCl (pH 8.0) to remove the residual medium, and the outer cell membrane was removed by subjecting the cells to osmotic shock through rotation in a solution of 1 mL of STE [0.5 M sucrose, 10 Mm Tris-HCl, 10 Mm EDTA (pH 8.0)] at 37°C for 30 minutes. A mixture of 1 mL of solution A and 20 µL of 50 mg/mL lysozyme solution was rotated at 37°C for 15 minutes to remove the peptidoglycan layer. After washing with 1 mL of PBS, 300 µL of the solution was mixed with 700 µL of PBS and 200 nM SMO-CRD-GST-Alexa 488 probe (based on monomers) was added and rotated at room temperature for 1 hour to label the spheroplasts with the fluorescent probe. Clones showing high fluorescence due to increased antigen binding ability were recovered by flow cytometry. From the recovered clones, scFv genes were amplified by PCR, and the process of sorting spheroplasts exhibiting high affinity to the SMO CRD region through culture, expression induction, spheroplasting for removing the outer cell membrane and peptidoglycan layer, antigen labeling, and selective gating by flow cytometry was repeated. After the sorting process by flow cytometry and a resorting process to increase sorting purity, followed by an enrichment process, a process of securing the sorted scFv variant genes through PCR amplification, a subcloning process, and a transformation process, the next round of sorting process was performed to enrich the desired clones. Through repeated rounds of search, the scFv variant clones with improved binding to the SMO CRD region were obtained (FIG. 9, FACS data). Their CDR and FR sequences are shown in Tables 1 and 2.

**[Table 1]**

| | CDR sequences of screened antibodies | | | | | |
|---|---|---|---|---|---|---|
| Antibody | CDRH1 | CDRH2 | CDRH3 | CDRL1 | CDRL2 | CDRL3 |
| #40 | GFAIGDNA (SEQ ID NO: 1) | IRSNSYGGTA (SEQ ID NO: 2) | ARRAHYYGGSTYNPS WYFDL (SEQ ID NO: 3) | QDISRR (SEQ ID NO: 4) | GAS (SEQ ID NO: 5) | QQGYSSPRT (SEQ ID NO: 6) |
| #40-4 | Same as #40 | Same as #40 | ARRAHYLGGSTYNPS WYFDL (SEQ ID NO: 7) | Same as #40 | Same as #40 | Same as #40 |
| #40-16 | Same as #40 | Same as #40 | ARRAHYYGGSNYNPS WYFDL (SEQ ID NO: 8) | Same as #40 | Same as #40 | Same as #40 |
| #40-22 | Same as #40 | Same as #40 | ARRAHYYGGSTYNPS WYVDL (SEQ ID NO: 9) | Same as #40 | Same as #40 | Same as #40 |
| #47 | Same as #40 | Same as #40 | ARRAHYYDGSTYNPS WYFDL (SEQ ID NO: 10) | Same as #40 | Same as #40 | Same as #40 |
| #47-7 | Same as #40 | Same as #40 | ARRAHHYGGSTYNPS WYFDL (SEQ ID NO: 11) | Same as #40 | Same as #40 | Same as #40 |
| #47-14 | Same as #40 | Same as #40 | ARRAHYYGGSTFNPS WYFDL (SEQ ID NO: 12) | Same as #40 | Same as #40 | QQGYSPPRT (SEQ ID NO: 13) |
| #42 | GYTFASYD (SEQ ID NO: 14) | INPAGGST (SEQ ID NO: 15) | ARTPSQTYELDY (SEQ ID NO: 16) | QGIRND (SEQ ID NO: 17) | AAS (SEQ ID NO: 18) | LQSFSTPYT (SEQ ID NO: 19) |

**[Table 2]**

| | FR sequences of screened antibodies | | | | | | | |
|---|---|---|---|---|---|---|---|---|
| Antibody | FRH1 | FRH2 | FRH3 | FRH4 | FRL1 | FRL2 | FRL3 | FRL4 |
| #40 | QVQLVQ SGGGVV QPGSSL RLSCEAS (SEQ ID NO: 20) | LNWVRQ APGKGLE WVGY (SEQ ID NO: 21) | EYAASVKG RFTISRDE SKNIAYLE MNSLRAE DTAVYYC (SEQ ID NO: 22) | WGRGTP VTVSS (SEQ ID NO: 23) | EIVLTQS PSFLSAS VGDRVTI TCRAS (SEQ ID NO: 24) | LAWYQR KPGKAPK LLIY (SEQ ID NO: 25) | NLASGVPS RFTGSGS GTEFTLTIS SLQSDDFA TYYC (SEQ ID NO: 26) | FGQGTK VDIKR (SEQ ID NO: 27) |
| #40-4 | Same as #40 | Same as #40 | Same as #40 | Same as #40 | Same as #40 | Same as #40 | Same as #40 | Same as #40 |
| #40-16 | Same as #40 | Same as #40 | Same as #40 | Same as #40 | Same as #40 | Same as #40 | Same as #40 | Same as #40 |
| #40-22 | Same as #40 | Same as #40 | Same as #40 | Same as #40 | Same as #40 | Same as #40 | Same as #40 | Same as #40 |
| #47 | Same as #40 | Same as #40 | EYAASVKG RFTISKDE SKNIAYLE MNSLRAE DTAVYYC (SEQ ID NO: 28) | WGRGTL VTVSS (SEQ ID NO: 29) | Same as #40 | LAWYQQ KPGKAPK LLIY (SEQ ID NO: 30) | NLASGVPS RFTGSGS GTEFTLTIS SLQPDDFA TYYC (SEQ ID NO: 31) | Same as #40 |
| #47-7 | Same as #40 | Same as #40 | Same as #47 | Same as #47 | Same as #40 | Same as #47 | Same as #47 | Same as #40 |
| #47-14 | Same as #40 | Same as #40 | Same as #47 | Same as #47 | Same as #40 | Same as #47 | Same as #47 | Same as #40 |
| #42 | QVRLVQ SGAEVKK PGASVKV SCRAS (SEQ ID NO: 32) | IHWVRQA PGQGPE WMGM (SEQ ID NO: 33) | LYAEGFEG RLTMTRDR SMSTAYM ELSSLRSE DTAVYYC (SEQ ID NO: 34) | WGQGTL VTVSS (SEQ ID NO: 35) | ETTLTQS PSSLSAS VGDRVTI TCRAS (SEQ ID NO: 36) | LAWYQQ KPGKAPK LLIY (SEQ ID NO: 37) | TLESGVPP RFSGSGS ETDFTLTLI RLQPDDFA TYYC (SEQ ID NO: 38) | FGQGTQ LTVLG (SEQ ID NO: 39) |

### <Example 10> Analysis of binding affinity of antibody variants with increased binding affinity to SMO CRD region by flow cytometry

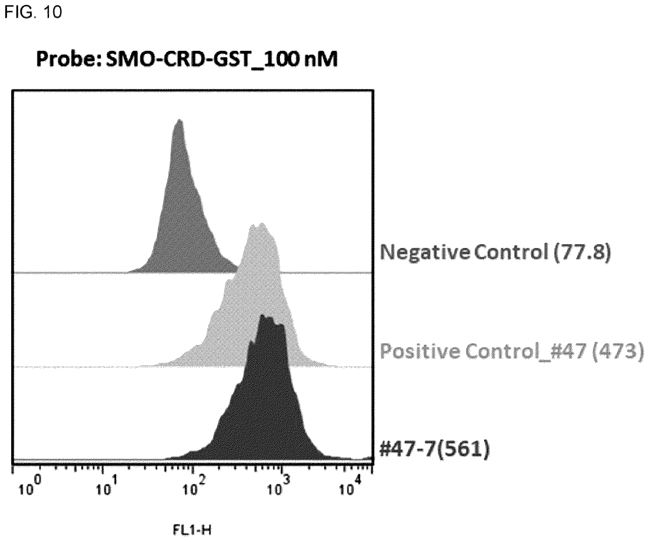

The binding affinity of the scFv antibody variants to the SMO CRD region was analyzed by flow cytometry. For this purpose, each variant and control were prepared from the spheroplasts using the method described above. The SMO CRD-GST-Alexa 488 antigen used for the screening was bound to the prepared spheroplasts at a concentration of 100 nM and incubated for 1 hour at room temperature. After washing twice with PBS to remove non-specific binding, the binding ability to the SMO CRD region was analyzed by flow cytometry. As a result of the analysis, it was confirmed that the scFv variant derived from the #47 antibody showed a significantly increased fluorescence signal as compared to the parent antibody #47 (FIG. 10).

### <Example 11> Production and purification of scFv antibody variant

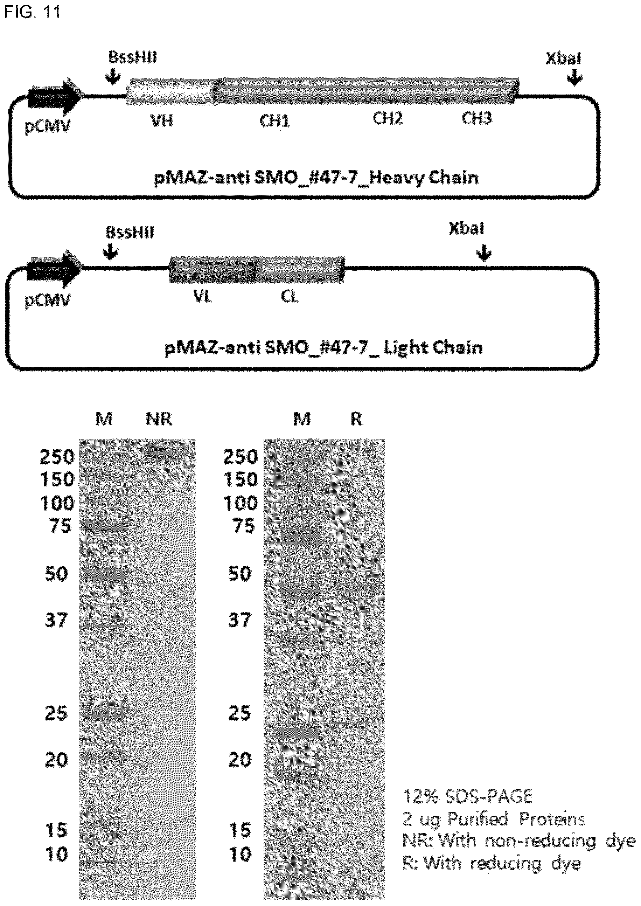

To confirm whether the binding affinity to SMO of the screened scFv antibody variant in IgG form was increased, scFv heavy chain and light chain expression vectors were prepared. Then, they were transfected into Expi293F animal cells. One day before the transfection, Expi293F cells were subcultured to a density of 2×10⁶ cells/mL, and transfected the next day using polyethylenimine (PEI, Polyscience, 23966). First, the variants were mixed in 100 mL of a Freestyle 293 expression culture medium (GIBCO, 12338-018), and then mixed with PEI:variant gene at a ratio of 4:1. After leaving at room temperature for 20 minutes, followed by mixing with the cells subcultured the previous day, the cells were cultured in a shaking CO₂ incubator at 37°C 125 rpm and 8% CO₂ for 7 days. The cells were centrifuged and only the supernatant was collected. Afterwards, equilibrium was achieved using 25x PBS. The culture was filtered with a 0.2-µm bottle top filter (Merck Millipore). 500 µL of protein A resin was added to the filtered culture medium, stirred at 4°C for 16 hours, flown through a column to recover the resin, washed with 5 mL of PBS, eluted with 3 mL of 100 mM glycine pH 2.7 buffer, and then neutralized using 1 M Tris-HCl pH 8.0. After exchanging the buffer with PBS using Centrifugal Filter Units 3K (Merck Millipore), the size and purity of each of the purified antibodies were analyzed by SDS-PAGE (FIG. 11).

### <Example 12> Analysis of binding affinity of antibodies with increased binding affinity to SMO

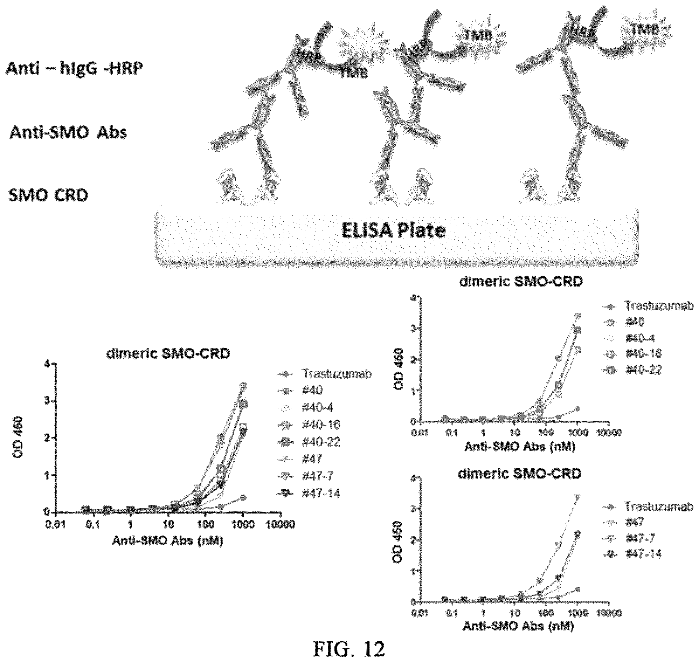

ELISA experiment was performed to measure whether the binding affinity of the antibodies to SMO was increased. 50 µL of dimeric SMO CRD fused with a GST tag at 4 µg/mL in 0.05 M Na₂CO₃ pH 9.6 was immobilized in a Flat Bottom Polystyrene High Bind 96-well microplate (Costar) at 4°C for 16 hours and then blocked by incubating at room temperature for 2 hours with 100 µL of 4% skim milk (GenomicBase) (in 0.05% PBST pH 6.0/pH 7.4). After washing 4 times with 180 µL of 0.05% PBST, 50 µL of the #47-7 antibody and the controls #40, #47 and trastuzumab serially diluted with 1% skim milk (in 0.05% PBST) were dispensed into each well and reacted for 1 hour at room temperature. After washing, antibody reaction was performed for 1 hour at room temperature using 50 µL of anti-human HRP conjugate (Jackson Immunoresearch), followed by washing. 50 µL of 1-Step Ultra TMB-ELISA Substrate solution (Thermo Fisher Scientific) was added to develop color, and the reaction was terminated by adding 50 µL of 2 M H₂SO₄, followed by analysis using an Epoch microplate spectrophotometer (BioTek) (FIG. 12).

### <Example 13> Confirmation of fully human SMO antibody binding with improved binding affinity in HCT 116 colon cancer cell line

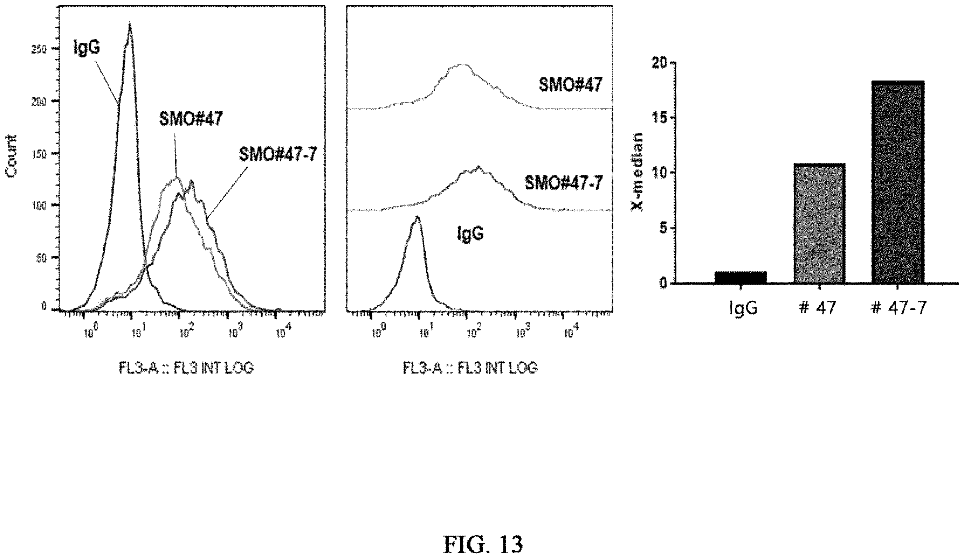

The binding affinity of the SMO-binding antibodies in HCT116 colon cancer cells was analyzed by flow cytometry. After blocking the HCT116 cells with BSA for 30 minutes, 10 µg of the SMO-binding antibody was bound and incubated for 1 hour at room temperature. One hour later, the cells were washed with PBS, reacted with 2 µL of Alexa 594 secondary antibody, and then incubated for 1 hour at room temperature. One hour later, the cells were washed with PBS to remove non-specific binding, and the binding ability of the SMO-binding antibodies was analyzed by flow cytometry. As a result of the analysis, it was confirmed that #47-7, which had improved binding affinity, had increased binding affinity to SMO in the HCT116 cells as compared to #47 (FIG. 13).

### <Example 14> Confirmation of ability of SMO receptor-binding antibody to inhibit hedgehog signaling activated by SHH

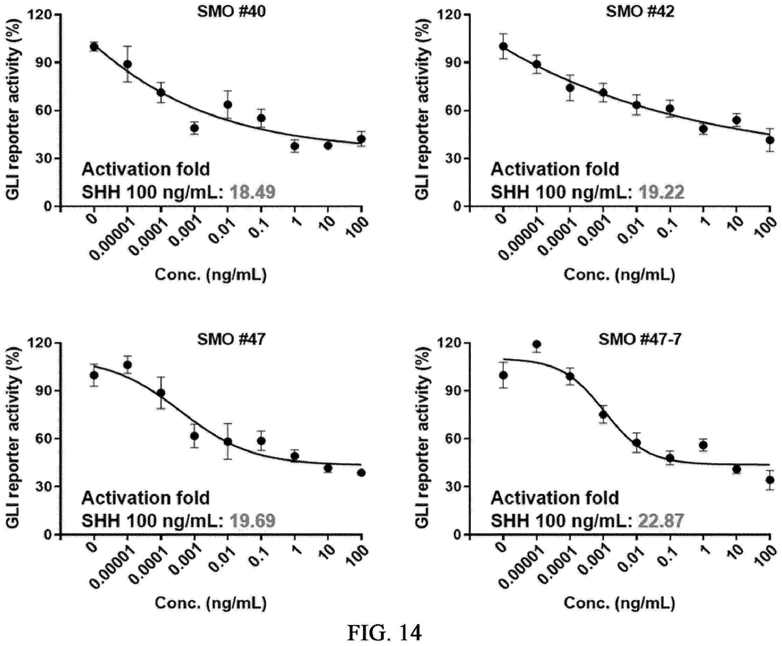

To confirm whether the antibodies of the present disclosure inhibit hedgehog signaling, experiment was performed as follows. In hedgehog signaling, signals are transmitted to PTCH and SMO receptors by hedgehog ligands, and the GLI protein, which is a transcription factor in the final stage, regulates the expression of various genes. To confirm this signaling, cell-based assay was performed in NIH3T3 cells containing a reporter plasmid with luciferase bound to the GLI protein-binding site. The NIH3T3 GLI cells were seeded in a 96-well plate at a density of 2.5x10⁴ cells per well, and treated the next day with the SMO-binding antibody diluted to 1/10 at a maximum concentration of 100 ng/mL. One hour after the antibody treatment, the cells were treated with 100 ng/mL of the SHH ligand. 24 hours after the antibody treatment, luciferase activity was measured using a Promega kit. As a result of the measurement, the ability of the four scFv variants to inhibit GLI transcriptional activity was confirmed (FIG. 14).

### <Example 15> Confirmation of ability to SMO receptor-binding antibody inhibit hedgehog signaling activated by hydroxysterol 20(s)

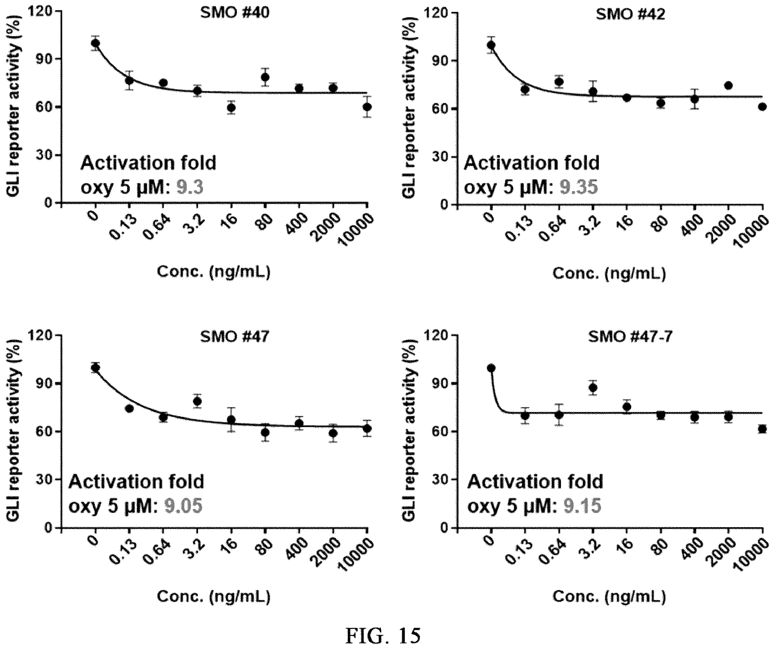

The ability of the SMO-binding antibody to inhibit GLI transcriptional activity was confirmed using the NIH3T3 GLI cells. NIH3T3 GLI cells were seeded in a 96-well plate at a density of 2.5×10⁴ cells per well and treated the next day with the SMO-binding antibody diluted to 1/5 at the highest concentration of 10,000 ng/mL. One hour after the antibody treatment, the cells were treated with 10 µM hydroxycholesterol 20(s). 24 hours after the antibody treatment, luciferase activity was measured using a Promega kit. As a result of the measurement, the ability of the four scFv variants to inhibit GLI transcriptional activity was confirmed (FIG. 15).

### <Example 16> Confirmation of ability of SMO antibodies (#40, #42, #47 and #47-7) to inhibit mobility of SKOV3 cancer cells

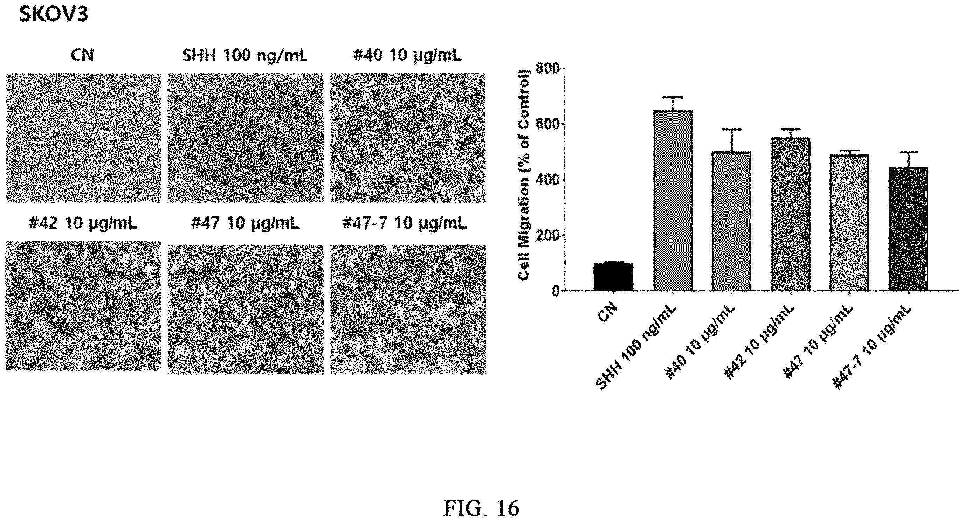

The ability of the SMO antibodies to inhibit cancer cell mobility was confirmed using SKOV3 cancer cells. SKOV3 (5×10⁴) cells were treated with the antibody at a concentration of 10 µg/mL for 30 minutes and then seeded in an upper chamber of a transwell. A lower chamber was treated with the SHH peptide ligand at 100 ng/mL. 48 hours later, the transwell was stained using Diff-Quick. As a result of the staining, it was confirmed that most of the antibodies inhibited cancer cell mobility by 20-30% and, among them, #47-7 with improved binding ability showed the best ability of inhibiting cancer cell mobility as 37% (FIG. 16).

Although the exemplary embodiments of the present disclosure have been described above, those skilled in the art can add, change or delete elements without departing from the spirit of the present disclosure as set forth in the patent claims. The present disclosure may be modified and changed in various ways, which will also be included within the scope of the present disclosure.

## Claims

1. A monoclonal antibody or an antigen-binding fragment thereof that recognizes the SMO (smoothened) protein as an antigen and binds specifically thereto, wherein the monoclonal antibody or the antigen-binding fragment comprises the following CDR region:
(a) CDRH1 of SEQ ID NO: 1;
(b) CDRH2 of SEQ ID NO: 2;
(c) CDRH3 of SEQ ID NO: 3, or CDRH3 containing an amino acid in which the amino acid at a position selected from a group consisting of the 6th, 7th, 8th, 11th, 12th and 18th positions in SEQ ID NO: 3 is substituted with another amino acid;
(d) CDRL1 of SEQ ID NO: 4;
(e) CDRL2 of SEQ ID NO: 5; and
(f) CDRL3 of SEQ ID NO: 6, or CDRL3 containing an amino acid in which the amino acid at the 6th position in SEQ ID NO: 6 is substituted with another amino acid.

2. The monoclonal antibody or the antigen-binding fragment thereof according to claim 1, wherein the 6th amino acid of SEQ ID NO: 3 is substituted from Y to H.

3. The monoclonal antibody or the antigen-binding fragment thereof according to claim 1, wherein the 7th amino acid of SEQ ID NO: 3 is substituted from Y to L.

4. The monoclonal antibody or the antigen-binding fragment thereof according to claim 1, wherein the 8th amino acid of SEQ ID NO: 3 is substituted from G to D.

5. The monoclonal antibody or the antigen-binding fragment thereof according to claim 1, wherein the 11th amino acid of SEQ ID NO: 3 is substituted from T to N.

6. The monoclonal antibody the antigen-binding fragment thereof according to claim 1, wherein the 12th amino acid of SEQ ID NO: 3 is substituted from Y to F.

7. The monoclonal antibody or the antigen-binding fragment thereof according to claim 1, wherein the 18th amino acid of SEQ ID NO: 3 is substituted from F to V.

8. The monoclonal antibody or the antigen-binding fragment thereof according to claim 1, wherein the 6th amino acid of SEQ ID NO: 6 is substituted from S to P.

9. The monoclonal antibody or the antigen-binding fragment thereof according to claim 1, wherein the CDRH3 is composed of an amino acid sequence selected from a group consisting of amino acid sequences of SEQ ID NO: 3, SEQ ID NO: 7, SEQ ID NO: 8, SEQ ID NO: 9, SEQ ID NO: 10, SEQ ID NO: 11 and SEQ ID NO: 12.

10. The monoclonal antibody or the antigen-binding fragment thereof according to claim 1, wherein the CDRL3 is composed of an amino acid sequence selected from a group consisting of amino acid sequences of SEQ ID NO: 6 and SEQ ID NO: 13.

11. A monoclonal antibody or an antigen-binding fragment thereof that recognizes the SMO (smoothened) protein as an antigen and binds specifically thereto, comprising CDRH1 of SEQ ID NO: 1; CDRH2 of SEQ ID NO: 2; CDRH3 having an amino acid sequence represented by the following General Formula 1; CDRL1 of SEQ ID NO: 4; CDRL2 of SEQ ID NO: 5; and CDRL3 having an amino acid sequence represented by the following General Formula 2:
**General Formula 1** A-R-R-A-H-**X1**-**X2**-**X3**-G-S-**X4**-**X5**-N-P-S-W-Y-**X6**-D-L;
**General Formula 2** Q-Q-G-Y-S-**X7**-P-R-T;
wherein, in General Formula 1, X1 is Y or H, X2 is Y or L, X3 is G or D, X4 is T or N, X5 is Y or F, and X6 is F or V, and, in General Formula 2, X7 is S or P.

12. A monoclonal antibody or an antigen-binding fragment thereof that recognizes the SMO (smoothened) protein as an antigen and binds specifically thereto, comprising CDRH1 of SEQ ID NO: 14; CDRH2 of SEQ ID NO: 15; CDRH3 of SEQ ID NO: 16; CDRL1 of SEQ ID NO: 17; CDRL2 of SEQ ID NO: 18; and CDRL3 of SEQ ID NO: 19.

13. A nucleic acid molecule encoding the monoclonal antibody or the antigen-binding fragment thereof according to claim 1, 11 or 12.

14. A vector comprising the nucleic acid molecule according to claim 13.

15. A host cell comprising the vector according to claim 14.

16. A pharmaceutical composition for preventing or treating cancer, comprising the monoclonal antibody or the antigen-binding fragment thereof according to claim 1, 11 or 12, a nucleic acid molecule encoding the monoclonal antibody or the antigen-binding fragment thereof, or a vector comprising the nucleic acid molecule.

17. A method for preventing or treating cancer, comprising a step of administering a therapeutically effective amount of the monoclonal antibody or the antigen-binding fragment thereof according to claim 1, 11 or 12, a nucleic acid molecule encoding the monoclonal antibody or the antigen-binding fragment thereof, or a vector comprising the nucleic acid molecule a to subject in need thereof.

18. A therapeutic use of the monoclonal antibody or the antigen-binding fragment thereof according to claim 1, 11 or 12, a nucleic acid molecule encoding the monoclonal antibody or the antigen-binding fragment thereof, or a vector containing the nucleic acid molecule.

19. The therapeutic use according to claim 18, wherein the therapeutic use is for treatment of cancer.

20. A method for quantifying the SMO (smoothened) protein comprised in a sample, comprising a step of treating a sample isolated from a subject with the monoclonal antibody or the antigen-binding fragment thereof according to claim 1, 11, or 12.

21. A method for providing information for diagnosis of a disease caused by overexpression of the SMO (smoothened) protein, comprising:
(a) a step of obtaining a sample isolated from a subject;
(b) a step of treating the sample with the monoclonal antibody or the antigen-binding fragment thereof according to claim 1, 11, or 12; and
(c) a step of confirming whether the expression level of the SMO protein comprised in the subject's sample is higher than the expression level of the SMO protein comprised in a normal group sample.

22. The method according to claim 21, wherein the disease caused by overexpression of the SMO protein is cancer.

23. A SMO (smoothened) protein quantitation kit comprising the monoclonal antibody or the antigen-binding fragment thereof according to claim 1, 11 or 12.

24. A composition for diagnosing cancer, comprising the monoclonal antibody or the antigen-binding fragment thereof according to claim 1, 11 or 12.
